## Europäisches Patentamt
## European Patent Office
## Office européen des brevets

(19)

(11) Veröffentlichungsnummer: **0 465 459 B1**

(12)
# EUROPÄISCHE PATENTSCHRIFT

(45) Veröffentlichungstag der Patentschrift: **06.12.95**

(51) Int. Cl.6: **A61H 39/00**, A61N 5/06, H01S 3/133, H01H 27/00

(21) Anmeldenummer: **91890135.6**

(22) Anmeldetag: **28.06.91**

Teilanmeldung 95100001.7 eingereicht am 28/06/91.

(54) **Miniaturlaser für human- und veterinärmedizinische Anwendung.**

(30) Priorität: **29.06.90 AT 1388/90**
**28.05.91 AT 1074/91**

(43) Veröffentlichungstag der Anmeldung:
**08.01.92 Patentblatt 92/02**

(45) Bekanntmachung des Hinweises auf die Patenterteilung:
**06.12.95 Patentblatt 95/49**

(84) Benannte Vertragsstaaten:
**AT BE CH DE DK ES FR GB GR IT LI LU NL SE**

(56) Entgegenhaltungen:
EP-A- 0 074 914
DE-A- 3 226 507
DE-A- 3 719 561
DE-U- 8 911 606
FR-A- 2 420 352

PATENT ABSTRACTS OF JAPAN, Band 13, Nr. 313 (C-618), 17. Juli 1989; & JP-A-1 099 575 (MATSUSHITA ELECTRIC IND. CO., LTD) 18- 04-1989

(73) Patentinhaber: **Walter, Helmut Dipl.-Ing.Dr.**
**Ferdinand-Andristrasse 2**
**A-3340 Waidhofen/Ybbs (AT)**

(72) Erfinder: **Walter, Helmut Dipl.-Ing.Dr.**
**Ferdinand-Andristrasse 2**
**A-3340 Waidhofen/Ybbs (AT)**

(74) Vertreter: **Casati, Wilhelm, Dipl.-Ing. et al**
**Patentanwälte Casati, Wilhelm, Dipl.-Ing.**
**Itze, Peter, Dipl.-Ing.**
**Amerlingstrasse 8**
**A-1061 Wien (AT)**

Rank Xerox (UK) Business Services
(3.10/3.09/3.3.3)

PATENT ABSTRACTS OF JAPAN, Band 13, Nr. 313 (C-618), 17. Juli 1989; & JP-A-1 099 573 (MATSUHITA ELECTRIC IND. CO., LTD) 18-04- 1989

IBM TECHNICAL DISCLOSURE BULLETIN, Band 11, Nr. 10, März 1969, Seite 1311, IBM Corp., New York, US; P. ABRAMSON: "On-off Switch"

PATENT ABSTRACTS OF JAPAN, Band 13, Nr. 235 (E-766), 30. Mai 1989; & JP-A-1 039 839 (NEC CORP.) 10-02-1989

PATENT ABSTRACTS OF JAPAN, Band 11, Nr. 227 (E-526)[2674], 23. Juli 1987; & JP-A-62 043 190 (SANYO ELECTRIC CO., LTD) 25-02-1987

## Beschreibung

Die Erfindung betrifft einen Miniaturlaser für human- und veterinärmedizinische Anwendung, insbesondere für Akupunktur und Wundbehandlung, mit einem Gehäuse, in dem die Steuereinrichtung für den Laser angeordnet ist, wobei auf dem Kopf des Gehäuses verschiedene Objektive auswechselbar anordenbar sind.

Bekannt sind tragbare batteriebetriebene Laserbestrahlgeräte, die entweder mit Infrarotlaserdioden oder Laserdioden, die im sichtbaren Wellenlängenbereich abstrahlen, bestückt sind, und deren divergenter Strahl direkt, oder mit einer Sammellinse fokussiert, auf die Hautoberfläche einwirkt.

Der Nachteil von Infrarotlaserbestrahlgeräten liegt zum einen in der Unsichtbarkeit der Infrarotbestrahlung, die eine visuelle Kontrolle der Behandlung für Therapeut und Patient ausschließt, und zum anderem in dem noch umstrittenen Wirkungsmechanismus der Infrarotstrahlung.

Da die von Laserdioden abgegebene Lichtleistung mit zunehmender Temperatur sinkt, müssen sie mit der auf demselben Chip befindlichen Photodiode in einer elektronischen Regelschaltung betrieben werden, die die abgegebene Lichtleistung konstant hält.

Alle diese Laserbestrahlgeräte haben zum einen den Nachteil, daß das Strahlprofil des Laserstrahls fest vorgegeben ist (siehe z.B DE-A-3 719 561) und nicht, wie in verschiedenen medizinischen Anwendungsgebieten erforderlich, verändert werden kann, und sie zum anderen mit sehr aufwendigen und damit teuren Regelschaltungen versehen sind.

Durch die DE-OS 32 26 507 wurde ein Miniaturlaser für die therapeutische Behandlung bekannt, der ein Gehäuse besitzt, in dem die Steuerung für die Laser-Diode angeordnet ist, wobei auf den Kopf des Gehäuses unterschiedliche Objektive aufgesteckt werden können. Zwei derartige Objektive sind dargestellt, wobei eines ein Parallel-Strahlenbündel liefert, das andere hingegen einen Cross-Over vor dem Objektiv erzeugt. Die Laser-Diode befindet sich bei dem bekannten Gerät vorne am Kopf des Gehäuses und ist damit allen mechanischen Einflüssen ungeschützt ausgeliefert. Bei dem bekannten Gerät tritt das Problem der Kollimation des Laserstrahles auf. Bekanntermaßen und wie dies auch aus der genannten Druckschrift hervorgeht, strahlt die Laser-Diode Licht als stark divergentes Strahlenbündel mit ellipsenförmigem Querschnitt ab. Um nun die Einkoppelungsverluste möglichst klein zu halten, sind zur Kollimation des Strahles Linsen mit extrem kurzen Brennweiten erforderlich, da es hiedurch gelingt, die numerische Apertur (i.e. der Sinus des halben Fangwinkels der Linse, wobei der Tangens des halben Fangwinkels definiert ist als Quotient aus Linsenradius und Entfernung des Brennpunktes der Linse bzw. der Laserdiode von der Mittenebene der Kollimationslinse) groß zu halten. Durch das aus der DE-OS 32 26 507 bekanntgewordene Aufstecken einer Objektivhülse auf das Gehäuse der Laser-Diode ist eine gute Bündelung des Laserstrahles nicht erreichbar, weil weder die Gehäuseabmessungen der Laser-Diode noch die genaue Lage des die Laserstrahlung erzeugenden Halbleiter-Plättchens innerhalb des Gehäuses spezifiziert sind. Durch das Aufstecken ist darüberhinaus eine genaue axiale Anordnung des jeweiligen Objektivs in den Strahlengang nicht möglich. Eine Stabilität gegen Erschütterungen, wie sie im Zuge einer Therapie auftreten können, ist bei der bekannten Anordnung nicht gegeben.

Aufgabe der Erfindung ist es, primär eine optimale Parallelrichtung des Laserstrahls zu erreichen. In einfachster Weise schlägt die Erfindung vor, die Laser-Diode in Richtung der optischen Achse verstellbar im Gehäuse anzuordnen. Dabei gelingt es, die Laserdiode bis auf 1/100 - Millimeter genau in den Brennpunkt der Kollimationslinse zu justieren und damit eine optimale Parällelrichtung des Laserstrahls sicherzustellen. Darüberhinaus wird hiedurch die Voraussetzung geschaffen, die Form des Strahlquerschnittes mit Wechselobjektiven beliebig einstellen zu können, weil von einem homogenen, parallelen kreisförmigen Laserstrahl ausgegangen werden kann. Die Randsthahlung des im Querschnitt schlank elliptischen Lichtkegels der Laserdüse kann durch eine Blende ausgeblendet werden, sodaß ein kreisförmiger Strahlquerschnitt überbleibt. Die Blendenwirkung kann bei dem erfindungsgemäßen Gerät durch entsprechende Gestaltung der Weite der Bohrungen im Laserkopf und den Wechselobjektiven realisiert werden. Da ein paralleler Grundstrahl vorliegt, ist die Entfernung eines Wechselobjektives vom Laserkopf unwesentlich, wodurch man in der Objektivberechnung einen Freiheitsgrad erhält. Da durch die erfindungsgemäße Maßnahme ein Laserstrahl den Laserkopf bereits gebündelt verläßt, erspart man sich, jedes einzelne der Wechselobjektive mit einer gesonderten Kollimationslinse auszustatten. Dadurch wird einerseits eine Einsparung an Kollimationslinsen erzielt, da nur eine einzige Kollimationslinse erforderlich ist, und anderseits entfällt der sonst bei jedem Wechsel des Objektivs auszuführende Justiervorgang.

Der erfindungsgemäße Miniaturlaser kann etwa in der Größe eines Kugelschreibers hergestellt werden und arbeitet im sichtbaren Wellenlängenbereich (etwa von 632 nm bis 700 nm). Die Laserdiode wird mittels einer bauteileoptimierten Schaltung betrieben, womit die kleine Bauform erreicht werden kann, ebenso auch ein optimaler Schutz für neueste Laserdioden erzielt wird, die zwar höchste Lichtausbeuten besitzen, gleichzeitig aber sehr empfindlich sind. Die Geometrie des erfindungsgemäß erzielbaren parallelen Strahles kann durch nachträgliches Aufschrauben verschiedener Objektive auf den Kopf des Miniaturlasers verändert werden.

Die erfindungsgemäße Ausführungsform stellt schon mit dem Grundgerät einen optimal gebündelten parallelen Laserstrahl zur Verfügung, mit dem auch schlecht zugängliche Akupunkturpunkte (z.B. in der Mundhöhle) gereizt werden können, anderseits kann durch Aufschrauben einer Zerstreuungsoptik auf das Grundgerät auch die für die großflächige Haut- bzw. Wundbestrahlung erforderliche divergente Strahlung erzeugt werden.

In besonderer Ausgestaltung der Erfindung ist dabei die Laserdiode in einer Hülse fixierbar und in dieser durch ein Gewinde axial verstellbar angeordnet, wobei in dieser Hülse die Kollimationslinse fixiert ist. Dadurch ist es möglich, in besonders einfacher Weise die Laserdiode mit großer Genauigkeit in den Brennpunkt der Kollimationslinse zu bewegen, und diese Justierung auch dann im Betrieb beizubehalten. Die Laserdiode ist bei der erfindungsgemäßen Ausbildung vor mechanischen Beschädigungen in jeder Hinsicht geschützt. So kann z.B. das die Laserdiode vor Umgebungseinflüssen schützende Deckglas der Laserdiode weder zerstört noch zerkratzt werden. Kratzspuren sind deshalb sehr störend, da sie zum einen auf der Bestrahlfläche vergrößert abgebildet werden (Laserstrahlen werden an den Kanten gebrochen) und zum anderen die Oberflächenvergütung des Deckglases beschädigt wird, wodurch die Transmission für die Laserstrahlung herabgesetzt wird. Durch die spezielle Anordnung von Laserdiode und Kollimationslinse in der Hülse ist der Raum zwischen Laserdiode und Kollimationslinse nach dem Justieren staubdicht abgeschlossen, sodaß ein Eindringen von Staubteilchen bzw. eine Verschmutzung der optischen Flächen ausgeschlossen werden kann, was deshalb von Bedeutung ist, weil aufgrund der stark divergenten Abstrahleigenschaften der Laserdiode Staubpartikel und andere Verunreinigungen auf der Hautoberfläche des Patienten vergrößert abgebildet werden, wodurch die Strahlung inhomogen wird, bzw. dunkle Flecken entstehen.

Wie bereits erwähnt, finden beim Erfindungsgegenstand vor allem Laserdioden Verwendung, die eine hohe Lichtausbeute ermöglichen, die jedoch gegen Überspannungen empfindlich sind. Ein weiterer Aspekt der Erfindung ist daher in Schaltungsmaßnahmen gelegen, um die empfindliche und kostspielige Laserdiode gegen Überspannungen zu schützen. Die Laserdiode zerstörende Spannungsspitzen können durch Spannungsspitzen in der Versorgungsspannung und/oder deren Prellen des Tasters beim Ein- und Ausschalten des Miniaturlasers verursacht werden.

Ansteigen der Versorgungsspannung führt zu einem Ansteigen des Betriebsstromes der Laserdiode. Treten in der Versorgungsspannung Spannungsspitzen auf, die so groß sind, daß der Maximalstrom überschritten wird, so wird die Laserdiode zerstört. Dem hilft nun die Erfindung bei einem Miniaturlaser, dessen Laser-Diode mit einer elektronischen Regelschaltung betrieben wird, dadurch ab, daß der Schutzwiderstand der Laser-Diode mindestens zum Teil in den Ohmschen Widerstand eines Tiefpaßfilters gelegt ist, das an der Eingangsseite der Steuerschaltung für die Laser-Diode angeordnet ist. Das eingangsseitig der elektronischen Regelschaltung für die Laserdiode angeordnete Tiefpaßfilter filtert Spannungsspitzen aus. Eine starke Tiefpaßwirkung kommt nur dann zustande, wenn der Widerstand in der Versorgungsleitung groß ist. Wird dieser Widerstand aber groß dimensioniert, geht aufgrund des durch den Betriebsstrom der Regelschaltung an ihm verursachten Spannungsabfalles ein Teil der Versorgungsspannung verloren, wodurch eine zusätzliche Batterie und damit eine größere Baugröße notwendig wäre. Da aber nun zum Betrieb der Laserdiode in der Regelschaltung ohnedies ein Vorwiderstand erforderlich ist, der den Diodenstrom begrenzt, kann dieser Widerstand - wie erfindungsgemäß vorgeschlagen - aus der Regelschaltung zumindest zum Teil herausgezogen und in den Ohmschen Widerstand des Tiefpaßfilters verlegt werden.

Um eine Zerstörung des Lasers durch Prellen des Tasters zu vermeiden, wird in Weiterbildung der Erfindung vorgeschlagen, daß im Regelkreis der Laserdiode zwei Transistoren angeordnet sind, wobei der den Strom zur Laserdiode begrenzende erste Transistor mit seiner Basis an den Emitter des zweiten Transistors angeschlossen ist, über dessen Basis die Kollektor/Emitterspannung des ersten Transistors überwacht wird, wobei die Kollektor-Emitterstrecke des zweiten Transistors den parallel zur Kollektor-Emitterstrecke des zweiten Transistors geschalteten Kondensator entladet und daß weiters der Kollektor des zweiten Transistors elektrisch mit dem Emitter des ersten Transistors verbunden ist, der seinerseits an Masse angeschlossen ist. Durch diese Maßnahme wird sichergestellt, daß in jedem Zeitpunkt eines Ein- und Ausschaltvorganges, unabhängig von der Größe der gerade anliegenden Spannung, definierte, nicht die Laserdiode zerstörende Betriebszustände herrschen, so daß trotz des Umstandes, daß bei Betätigen des Tasters auch die Versorgungsspannung der Regelelektronik mitgeschaltet wird, und es durch Prellen des Tasters zu einem unerwünschten zweiten Ein- und Ausschaltvorgang kommt, die Laserdiode nicht zerstört wird.

Einrichtungen, welche Laserstrahlen mit mehr als 1 mW Ausgangsleistung erzeugen, jedoch auch andere elektrische Stromkreise, welche Verbraucher versorgen und die den menschlichen Körper schädigen können, sind durch mechanische oder elektrische Verriegelungen zu sichern, damit das Gerät von Unbefugten nicht in Betrieb genommen werden kann.

Bekannt ist es, zu diesem Zweck Schlüsselschalter, elektronische Codeschlösser oder Magnetkarten zu verwenden, bei denen der Stromkreis nur durch Personen geschlossen werden kann, die den passenden Schlüssel oder Magnetkarten besitzen oder den jeweiligen Code kennen.

Alle diese Sicherheitseinrichtungen haben den Nachteil, daß sie im Hinblick auf ihren Raumbedarf einer Miniaturisierung nicht zugänglich sind oder einen zusätzlichen elektronischen Schaltungsaufwand erforderlich machen, der ebenfalls wieder Platz beansprucht, die Kosten erhöht und einen höheren Stromverbrauch verursacht, der bei Batteriebetrieb besonders nachteilig ist.

Außerdem hindern in einschlägigen Vorschriften enthaltene Zusatzforderungen, wie z.B. daß die Entriegelungsvorrichtung in der "Ein-Stellung" nicht abziehbar sein darf, die Anwendung vieler der am Markt erhältlichen Baugruppen.

Aufgabe der weiteren Erfindung ist es, eine einfache Lösung des Sicherungsproblems elektrischer Stromkreise für die Versorgung von Verbrauchern anzugeben, insbesondere wenn eine Schädigung von Menschen, durch unbefugte Inbetriebnahme des Verbrauchers auftreten könnte, wie z.B. bei Lasern. Die Erfindung schlägt hiebei für eine Sicherheitseinrichtung für elektrische Stromkreise zur Versorgung von Verbrauchern, insbes. für Miniaturlaser der zuvor beschriebenen erfindungsgemäßen Gestaltung mit mindestens einem in den Stromkreis eingebauten Schalter vor, daß der Schalter von einem elektrisch leitenden Stift od.dgl., gebildet ist, der durch einen Schlitz in einem elektrisch leitenden, den Verbraucher aufweisenden Gehäuse einführbar ist, wobei im Weg des Stiftes ein mit dem Verbraucher elektrisch verbundener Kontakt gelegen ist. Durch diese Maßnahme wird der Stromkreis durch Einführen des Stiftes geschlossen und bleibt solange geschlossen, als der Stift im Schlitz belassen wird. Nach Abziehen des Stiftes wird der Verbraucher sicher ausgeschaltet und ein neuerliches Einschalten ist nur durch Personen möglich, die den Stift besitzen.

In besonderer, weiterer Ausgestaltung der Erfindung ist der mit dem Verbraucher verbundene elektrische Kontakt, in an sich bekannter Weise von einem Pol einer, entgegen dem Druck einer Feder im Gehäuse verschiebbar gelagerten Batterie gebildet, für deren Abstützung ein aus elektrisch isolierendem Material bestehender Anschlag vorgesehen ist, an dem die Batterie unter dem Druck einer Feder bei aus dem Gehäuse entferntem Stift zur Anlage kommt. Diese Ausgestaltung ist besonders einfach, da die Batterie selbst für das Ein- und Ausschalten benutzt wird.

Der Stift kann auf besonders einfache Weise in der Einschaltstellung gehalten werden, wenn er vom Bart eines Schlüssels gebildet ist, der mit flachen Rippen versehen ist, die durch den Schlitz des Gehäuses hindurchführbar sind und nach Drehung an der Unterseite des den Schlitz aufweisenden Gehäuseteiles, der insbesondere als Schraubkappe ausgebildet ist, abstützbar sind.

In besonderer Ausgestaltung der erfindungsgemäßen Einrichtung ist der Anschlag aus isolierendem Material als Ring ausgebildet und in den, den Schlitz aufweisenden Gehäuseteil eingesetzt. Durch diese Ausgestaltung wird ein sicherer Sitz der Batterie in der abgeschalteten Stellung erzielt, da die Abstützung an der Stirnfläche der Batterie erfolgt.

Die Erfindung wird nachstehend anhand der Zeichnung, die beispielshaft erfindungswesentliche Details veranschaulicht, näher erläutert. Es zeigen,

Fig. 1 in einem teilweisen Längsschnitt einen erfindungsgemäß ausgebildeten Miniaturlaser,

Fig. 2 ein Schaltschema für eine Laserdiode, wie sie in einem Miniaturlaser gemäß Fig. 1 verwendet werden kann,

Fig. 3 einen Teil eines Miniaturlasers gemäß Fig. 1 in einem Längsschnitt, mit einer Sicherheitseinrichtung gegen unbefugte Inbetriebnahme,

Fig. 4 eine Ansicht auf den in Fig. 3 dargestellten Teil des Miniaturlasers in Richtung des Pfeiles IV in Fig. 3,

Fig. 5 einen der Fig. 3 entsprechenden Schnitt, jedoch in Einschaltstellung, in einem Schnitt gemäß Linie V-V in

Fig. 6, die eine Ansicht in Richtung des Pfeiles VI auf die Fig. 5 wiedergibt,

Fig. 7 einen Schnitt entlang der Linie VII-VII in Fig. 6, und

Fig. 8 schematisch den grundsätzlichen Aufbau einer Sicherheitseinrichtung gegen unbefugte Inbetriebnahme des Miniaturlasers.

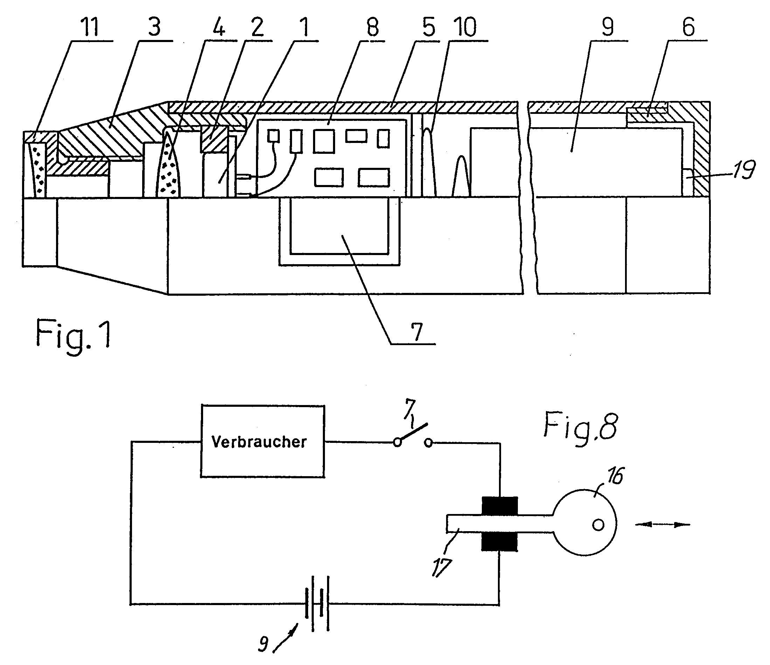

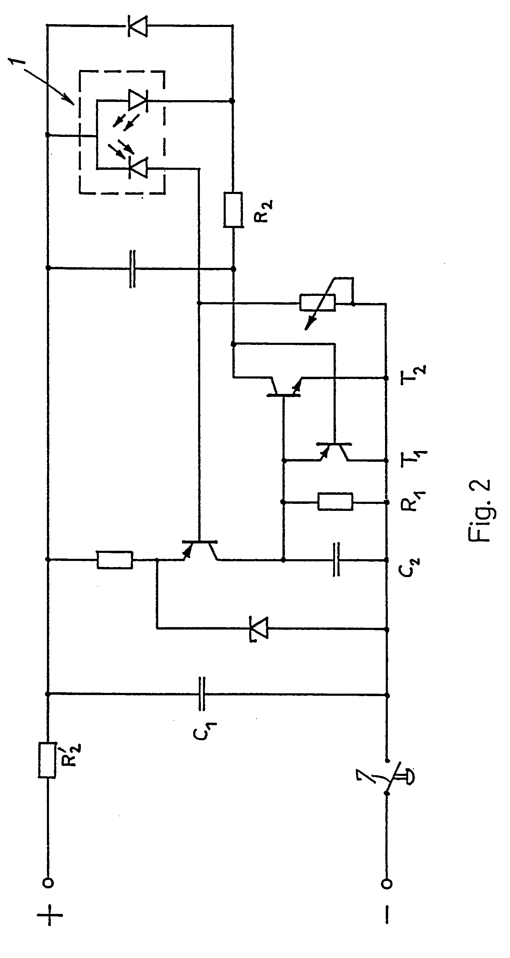

Die Laserdiode 1 ist in eine Gewindehülse 3 befestigt, insbesondere eingeklebt und kann in Axialrichtung der Gewindehülse 3, die den konischen Vorderteil des Gehäuses 5, 6 bildet, genau in den Brennpunkt der Sammellinse (Kollimationslinse) 4 gedreht werden, wo sie dann, bevorzugt durch einen Klebertropfen fixiert wird. Die Gewindehülse 3 des Miniaturlasers ist in den als Rohr 5 ausgebildeten Teil des Gehäuses eingepreßt, das die notwendige elektrische Verbindung zu einer Verschlußkappe 6 des Gehäuses herstellt. Mit 7 ist in Fig. 1 ein Ein-Austaster bezeichnet. Unterhalb des Ein/Aus-Tasters ist die in SMD-Technik gefertigte und in Fig. 2 in ihren erfindungswesentlichen Teilen dargestellte Regelschaltung 8 für die Laserdiode 1 angeordnet, die von Batterien 9 versorgt wird. Um einen guten elektrischen Kontakt zwischen den Batterien 9 und dem Gehäuse 5, 6 zu erzielen, ist eine Andruckfeder 10 vorgesehen, welche die Batterien 9 gegen die elektrisch leitende Verschlußkappe 6 drückt. Da alle Teile innerhalb der den konischen Vorderteil bildenden Hülse 3 aus einem leitfähigen Material angefertigt sind, ist durch diese Konstruktion gewährleistet, daß zum einen die Laserdiode 1 über das ganze Gehäuse 5, 6 gekühlt wird und zum anderen ein Minimum an Versorgungsleitungen für die Regelschaltung 8 erforderlich ist. Auf die Hülse 3, die den Kopf des Gehäuses bildet, werden Wechselobjektive 11 aufgeschraubt, die als Drehteile ausgeführt sind.

Die Kollimationsoptik 4 dient dazu, die divergente Strahlung der Laserdiode 1 in Richtung der optischen Achse des Lasers zu bündeln. Die Wechselobjektive 11 bedürfen demgemäß keiner weiteren Kollimationslinse.

Zum Betrieb der Laserdiode ist eine Regelschaltung vorgesehen, die in Fig. 2 dargestellt ist und die Laserdiode auch gegen Überstromspitzen schützt. Hiebei befindet sich auf der Eingangsseite der Regelschaltung ein Tiefpaßfilter, dessen Ohmscher Widerstand mit $R_2'$ und dessen Kondensator mit $C_1$ bezeichnet sind. Der Laserdiode 1 ist als Schutzwiderstand ein mit $R_2$ bezeichneter Widerstand vorgeschaltet. Eine starke Tiefpaßwirkung zur Ausfilterung von Spannungsspitzen kommt zustande, wenn der Widerstand in der Versorgungsleitung groß ist. Dies verursacht aber einen Verlust eines Teiles der Versorgungsspannung, bedingt durch den durch den Betriebsstrom der Regelschaltung am Widerstand verursachten Spannungsabfall. Da nun zum Betrieb der Laserdiode 1 in der Regelschaltung ein Vorwiderstand zur Begrenzung des Diodenstromes erforderlich ist, kann dieser Widerstand aus der Regelschaltung zumindest zum Teil herausgezogen und in den Ohmschen Widerstand des Tiefpaßfilters $R_2'$, $C_1$ verlegt werden.

Im Regelkreis der Laserdiode 1 sind weiters zwei Transistoren $T_1$ und $T_2$ angeordnet, wobei der den Strom zur Laserdiode 1 begrenzende erste Transistor $T_2$ mit seiner Basis an den Emitter des zweiten Transistors $T_1$ angeschlossen ist, über dessen Basis die Kollektor/Emitterspannung des ersten Transistors $T_2$ überwacht wird, wobei die Kollektor/Emitterstrecke des zweiten Transistors $T_1$ den parallel zur Kollektor-Emitterstrecke des zweiten Transistors $T_1$ geschalteten Condensator $C_2$ entladet und daß weiters der Kollektor des zweiten Transistors $T_1$ elektrisch mit dem Emitter des ersten Transistors $T_2$ verbunden ist, der seinerseits an Masse angeschlossen ist. Der parallel zur Kollektor-Emitterstrecke des zweiten Transistors $T_1$ geschaltete Kondensator $C_2$ ist dabei zu Beginn eines Prellvorganges (aufeinanderfolgendes Ein- und Ausschalten des Tasters 7) entladen, so daß erst nach dem relativ langsamen (einige Millisekunden dauernden) Wiederaufladevorgang des vorgenannten Kondensators $C_2$ der erste Transistor $T_2$ durchschaltet. Zu diesem Zeitpunkt ist die Photodiode aber schon aktiv und begrenzt über den Regelkreis den Strom der Laserdiode 1. Die Entladung des Kondensators $C_2$ bei Prellen des Tasters 7 kann durch Einbau des Entladewiderstandes $R_1$ beschleunigt werden. Dieser Entladewiderstand $R_1$ liegt parallel zur Kollektor-Emitterstrecke des zweiten Transistors $T_1$ und sorgt nach dem Ausschalten der Versorgungsspannung durch den Taster 7 für rasches Abfließen der Ladung des Kondensators $C_2$, damit bei Prellen des Tasters der Transistor $T_2$ nicht sofort, sondern erst nach erneuter Aufladung des Kondensators $C_2$ einschalten kann.

Um ein zu schnelles und niederohmiges Durchschalten des Transistors $T_2$ zu vermeiden, wird die CE-Spannung des Transistors $T_2$ mit dem zusätzlichen Transistor $T_1$ überwacht. $T_1$ wird genau dann leitend, wenn die CE-Spannung von $T_2$ zu stark abnimmt bzw. $T_2$ in Sättigung geht. Leitet nämlich $T_1$, so wird $C_2$ entladen, die BE-Spannung von $T_2$ verkleinert und der Laserdiodenstrom reduziert. Der Transistor $T_1$ begrenzt solcherart bei Prellen des Tasters 7 dynamisch den Strom durch die Laserdiode auf Werte unterhalb des Maximalstromes, durch Verhindern eines zu starken Absinkens der Kollektor-Emitterspannung des Transistors $T_2$, wodurch der Kollektorstrom von $T_2$, und damit der Strom durch die Laserdiode, begrenzt wird.

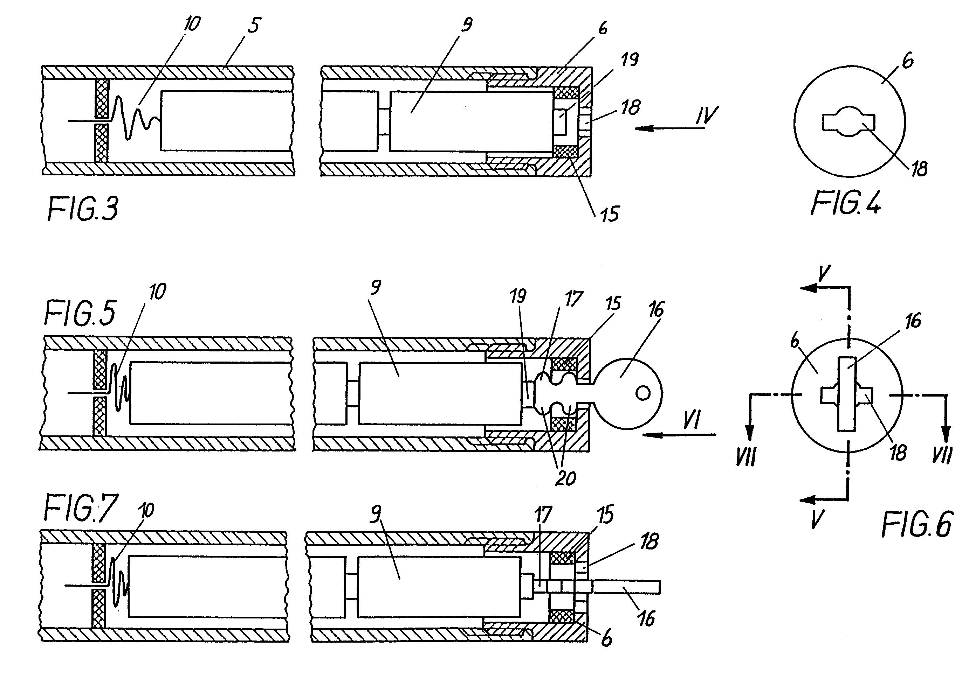

Bei der in den Fig. 3 - 8 dargestellten Sicherheitseinrichtung gegen unbefugtes Inbetriebnehmen des Miniaturlasers ist in den Stromkreis zur Versorgung des Verbrauchers (Lasers) mindestens ein Schalter eingebaut. Dieser ist von einem elektrisch leitenden Stift 17 gebildet, der durch einen Schlitz 18 in einem elektrisch leitenden, den Verbraucher aufweisenden Gehäuse 5, 6 einführbar ist, wobei im Weg des Stiftes 17 ein mit dem Verbraucher elektrisch verbundener Kontakt 19 gelegen ist. Dieser Kontakt ist von einem Pol einer, entgegen dem Druck einer Feder 10 im Gehäuse 5, 6 verschiebbar gelagerten Batterie 9 gebildet, für deren Abstützung ein aus elektrisch isolierendem Material bestehender Anschlag 15 vorgesehen ist, an dem die Batterie 9 unter dem Druck einer Feder 10 bei aus dem Gehäuse 5, 6 entferntem Stift 17 zur Anlage kommt. Der den Schalter bildende Stift 17 kann vom Bart eines Schlüssels 16 gebildet sein, der mit flachen Rippen 20 versehen ist, die durch den Schlitz 18 des Gehäuses 5, 6 hindurchführbar sind und nach Drehung an der Unterseite des den Schlitz 18 aufweisenden Gehäuseteiles 6, der insbesondere als Schraubkappe ausgebildet ist, abstützbar sind. Der Anschlag aus isolierendem Material, gegen den bei entferntem Stift 17 die Batterien 9 unter dem Druck der Andruckfeder 10 anliegen, kann als Ring ausgebildet sein. Der Ring ist bei dem dargestellten Ausführungsbeispiel in den, den Schlitz 18 aufweisenden Gehäuseteil (Verschlußkappe 6) eingesetzt und so dick ausgeführt, daß in der Aus-Stellung (Fig. 3) der der Andruckfeder 10 abgekehrte Pol (Kontakt 19) der äußeren der Batterien 9, die Metallfläche der Verschlußkappe 6 nicht berühren kann. Wird nun - ausgehend von dieser Stellung - der zum Schlüssel 16 gestaltete Stift 17 in den Schlitz 18 der Verschlußkappe 6 eingeführt, so drückt dieser Stift 17 auf den der Federseite abgewandten Pol der äußeren der Batterien 9 und stellt damit einen elektrischen Kontakt zu den Batterien her. Wird der Schlüssel 16 um 90° gedreht und anschließend an die Drehung losgelassen, so werden aufgrund der federnden Vorspannung der Batterien 9, die Rippen 20 des Schlüssels 16 gegen die leitfähige Rückwand der Abdeckkappe 6 gedrückt und ein elektrischer Kontakt zwischen den Batterien 9 und der Abdeckkappe 6, bzw. dem Gehäuse 5, 6, hergestellt. Der Schlüssel 16 kann nun, ohne den Kontakt wieder zu unterbrechen, nicht mehr abgezogen werden. Dieser Zustand entspricht der "Ein-Stellung" (Fig. 5 - 8). Wird der Schüssel 16 erneut um 90° gedreht, wird dieser von den federnd vorgespannten Batterien 9 ausgestoßen, die Batterien werden gegen den Anschlag 15 der Verschlußkappe 6 gepreßt, und die Verbindung zum Gehäuse 5, 6 unterbrochen (Fig. 3).

## Patentansprüche

1. Miniaturlaser für human- und veterinär-medizinische Anwendung, insbesondere für Akupunktur und Wundbehandlung, mit einer Laser-Diode und einem Gehäuse (5, 6), in dem die Steuereinrichtung (8) für den Laser (1) angeordnet ist, wobei auf den Kopf des Gehäuses (5, 6) verschiedene Objektive (11) auswechselbar anordenbar sind, wobei im Gehäuse (5, 6) eine Kollimationsoptik (4) angeordnet ist, um die divergente Strahlung der Laser-Diode (1) zu bündeln dadurch gekennzeichnet, daß die Laser-Diode (1) in Richtung der optischen Achse des Lasers verschiebbar und fixier bar im Gehäuse (5, 6) angeordnet ist und der Laserstahl den Laserkopf zu einem Parallelstrahl gebündelt verläßt.

2. Miniaturlaser nach Anspruch 1, dadurch gekennzeichnet, daß die Laserdiode (1) in einer Hülse (3) fixierbar ist und in dieser vor dem Fixieren durch ein Gewinde axial verstellbar angeordnet ist, wobei in dieser Hülse (3) die Kollimationslinse (4) fixiert ist.

## Claims

1. A miniature laser for use in human and veterinary medicine, particularly for acupuncture and the treatment of wounds, with a laser diode and a housing (5, 6) in which the control device (8) for the laser (1) is mounted, various lenses (11) being able to be interchangeably arranged onto the head of the housing (5, 6), and a collimation optical device (4) being mounted in the housing (5, 6) to focus the divergent radiation of the laser diode (1), characterized in that the laser diode (1) is displaceably and fixably mounted in the direction of the optical axis of the laser in the housing (5, 6), and the laser ray leaves the laser head focussed into a parallel ray.

2. A miniature laser according to claim 1, characterized in that the laser diode (1) is fixable in a bush (3), and is axially adjustably mounted in it by means of a screw thread, before fixing the collimation lens (4) being fixed in said bush (3).

## Revendications

1. Laser miniature destiné à être utilisé en médecine humaine et vétérinaire, notamment en acuponcture et pour le traitement de blessures, comportant une diode laser et un boîtier (5, 6) dans lequel est disposé le dispositif de commande (8) du laser (1), différents objectifs (11) pouvant être placés de façon interchangeable sur la tête du boîtier (5, 6), une optique de collimation (4) étant disposée dans le boîtier (5, 6) afin de focaliser le rayonnement divergent de la diode laser (1), caractérisé en ce que la diode laser (1) est disposée dans le boîtier (5, 6) de façon à être mobile en direction de l'axe optique et de façon à pouvoir être fixée et en ce que le rayon laser sort de la tête du laser rassemblé en un rayon parallèle.

2. Laser miniature selon la revendication 1, caractérisé en ce que la diode laser (1) peut être fixée dans un manchon (3) et que, avant la fixation, elle est disposée à l'intérieur de celui-ci de façon à pouvoir être déplacée axialement au moyen d'un filetage, la lentille de collimation (4) étant fixée dans ce manchon (3).

11  3  4  2  1  8  5  10  9  6

19

Fig.1

7

EP 0 465 459 B1

Verbraucher

7

Fig.8

16

17

9

Fig. 2

EP 0 465 459 B1

FIG.3

FIG.4

FIG.5

FIG.6

FIG.7

EP 0 465 459 B1